(19) Europäisches Patentamt
European Patent Office
Office européen des brevets

(11) **EP 4 606 309 A1**

(12) **EUROPEAN PATENT APPLICATION**

(43) Date of publication:
**27.08.2025 Bulletin 2025/35**

(21) Application number: **24158826.8**

(22) Date of filing: **21.02.2024**

(51) International Patent Classification (IPC):
*A61B 6/03* (2006.01)   *A61B 6/00* (2024.01)

(52) Cooperative Patent Classification (CPC):
**A61B 6/035; A61B 6/4441; A61B 6/4476;**
**A61B 6/56;** A61B 6/4405

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventors:
• **BODKHE, Sagar**
  **Eindhoven (NL)**
• **DE BOER, Jacob**
  **Eindhoven (NL)**
• **VERMA, Mahesh**
  **5656AG Eindhoven (NL)**

(74) Representative: **Philips Intellectual Property &**
**Standards**
**High Tech Campus 52**
**5656 AG Eindhoven (NL)**

(54) **MOTORIZED C-ARM SYSTEM**

(57)     The invention is about an X-ray system comprising a C-arc (102) provided with an X-ray source and an X-ray detector, and a carriage (101) of the C-arc (102) adapted to fixedly hold the C-arc (102) on the carriage (101). In addition, a drive arrangement to drive the rotation of the C-arc (102) about a main axis (1000) comprises a motor (10, 110) (with a rotor (11, 111) and a stator (12, 112)) and a transmission mechanism (20, 120) arranged to transmit the motion generated by the motor (10, 110) to the C-arc (102) rotating about the main axis (1000). The transmission mechanism (20, 120), having a predetermined ratio of transmission, comprises an input to which the rotor (11, 111) is mounted and an output to which the stator (12, 112) and the C-arc (102) are mounted.

FIG. 2A

FIG. 2B

**Description**

TECHNICAL FIELD

The present disclosure generally relates to the field of a medical imaging system, more particularly, to a motor assembly for a C-arm system.

BACKGROUND

C-arm is a type of medical imaging device commonly used in hospitals and medical centers and other medical facilities. A C-arm is an X-ray machine with a C-shaped frame ("C-arc") that allows the radiation source and the detector to rotate and align axially for different examinations and procedures. The C-arm is designed to provide high-quality images of bones, joints, and internal organs, and is particularly useful for surgical operations. It allows surgeons to visualize the surgical field during operations and obtain real-time images to guide their procedures.

A C-arm is designed to move in various directions, for example, to translate along horizontal or vertical directions or rotate about different axes. Also, the C-arm may be provided with different motorized assemblies to (semi-)automatically achieve the various motions. These assemblies should be compact to make the footprint of the C-arm as small as possible. In particular, it is desirable to have a driving system of the rotational movements of the C-arc as small as possible, especially because they are usually located closer to the core part of the C-arm system (i.e. the C-arc) than the driving system of the translational movements. These rotational movements may include a propelling movement (about a main horizontal axis virtually "splitting" the "C" or the C-arc mostly in two symmetrical parts) and/or an angular movement (about a transversal horizontal axis orthogonal to said main horizontal axis).

Said compactness is of a particular importance in mobile X-ray systems, which are more subject to collisions and used in environments having different sizes and configurations for different types of procedures, involving some manual use of the C-arm further to motorization, than fixed X-rays systems.

SUMMARY OF THE INVENTION

Exemplary embodiments of the present disclosure propose a solution to at least address said problems from the prior art.

In an embodiment, the disclosure is about an X-ray system comprising a C-arc provided with an X-ray source and an X-ray detector (or to be provided with an X-ray source and an X-ray detector), and a carriage (also called stand) of the C-arc adapted to fixedly hold the C-arc on the carriage. In addition, a drive arrangement, preferably arranged with or on the carriage or functionally arranged between the C-arc and the carriage, to drive the rotation of the C-arc about a main axis, comprises a motor (having a rotor and a stator) and a transmission mechanism arranged to transmit the motion generated by the motor to the C-arc rotating about the main axis. The transmission mechanism, having a predetermined ratio of transmission, comprises an input to which the rotor is mounted and an output to which the stator and the C-arc are mounted.

Mounting the stator (and indirectly the motor housing usually integral or assembled to the stator) to the transmission mechanism prevents the montage of the stator to the carriage (or carriage), such a montage of the motor to the carriage usually involving cumbersome fixation (e.g. screws) and damping elements. The arrangement according to this embodiment is therefore more compact. Another consequence of the montage of the stator to the output of the transmission mechanism results in the rotation of the stator together with the C-arc - which then requires an adaptation of the control of the motor accordingly. Another advantage is an easier maintenance of the C-arm system: for example, the motor may be replaced without having to dismount all the core elements of the propelling arrangement as explained more in detail in this disclosure.

In a particular embodiment, the transmission mechanism comprises a rotatable shaft mounted on the C-arc and on said output and rotatable about said main axis, and a fixed shaft mounted on the carriage so as to fixedly hold the transmission mechanism, so as to facilitate the propelling movement of the C-arc. Preferably the rotatable and fixed shafts are coaxial about the main axis, which further increases the compactness of the drive arrangement. In a particular case, the rotatable shaft has a lumen extending along the main axis, at least a part of the fixed shaft being arranged within this lumen. By this way the C-arc can be fixed on the free terminal part of the hollow shaft over a broader surface (which can be extended away from the main axis since the hollow shaft, as being the outer shaft element, has important surrounding free space) and away from the main axis. Optionally the fixed shaft is fixed to the carriage via a keyless bush, so as to make a torque applied to the fixed axis (especially in a manual mode) slipping away. Optionally the fixed shaft is held via an intermediate carrier on which a potentiometer is provided to, the potentiometer being engaged with the hollow shaft to measure angular positions. In a particular embodiment, a (spiral) electrical cable is provided (e.g. to supply motor and/or electromagnetic lock and/or some sensing elements - e.g. potentiometer, encoder - and receive power or data from these elements) within a gap between the rotatable shaft and the fixed shaft, further increasing the compactness of the drive arrangement.

In a particular embodiment, said transmission mechanism has a ratio of transmission (output / input) equal to or lower than 1/50, more particular equal to or lower than 1/100, more particular equal to or lower than 1/150, more particular around 1/160.

In a particular embodiment, the transmission mechanism comprises a harmonic drive comprising:

> a wave generator being the input,
> a circular-spline being the output,
> a flex-spline being mounted to the carriage, optionally via said fixed shaft if any.

It is known that a harmonic drive is a transmission mechanism comprising such three components resulting in compactness and light weight while having high gear ratio, addressing therefore above-mentioned needs. In addition, a harmonic drive has mostly no backlash meaning that the montage of the stator to the output will not involve disturbance or noise and a great positioning accuracy.

In a particular embodiment, the drive arrangement further comprises an electromagnetic lock arranged to lock the motor, optionally the electromagnetic lock being a drum brake, thus allowing an easy and remote lock/unlock of the motor (via e.g. a user interface provided beside or on the X-ray system). This can be useful to easily switch between a manual mode (i.e. the motor is locked, the whole motor block - rotor and stator - moves together as one rigid element, with the generated motion) and an automatic mode (i.e. the motor is free: the rotor can therefore generate the movement of the C-arc via the transmission mechanism). It is to be noted that, in the manual mode, the input being manually generated on the C-arc, said output of the transmission mechanism becomes the input and the input becomes the output which is locked. The drive arrangement according to the invention provides a reversing solution for easily switching between manual and automatic mode.

In a more particular case, the electromagnetic lock ("EM lock") is mounted on the side of the motor opposite said output of the transmission mechanism. With this specific side-by-side arrangement (EM lock - motor - transmission mechanism), one may obtain a generally elongated configuration, mostly a cylindrical one having a width close to the motor width resulting in a very compact configuration. Moreover the montage or fixation of the EM lock may be done on the yoke 103 in such a way that it faces the motor. By this way, this montage or fixation is not done on the carriage, preventing that such a montage of the EM lock to the carriage, the use of cumbersome fixation (e.g. screws) and damping elements in such a montage to the carriage. The arrangement according to this particular embodiment is therefore more compact. Another consequence of the montage of the EM lock to the yoke results in the rotation of the EM lock together with the stator together with the C-arc- since the stator is also mounted to the (output of the) transmission mechanism.

In a more particular case, the X-ray system further comprises a yoke directly mounted to or integral with the C-arc, wherein the drive arrangement is mounted to the yoke. Optionally at least another drive arrangement to drive other movement(s) of the C-arc (e.g. the angular movement) is embedded by the yoke.

In particular the carriage may be provided with wheels to be mobile with respect to the floor, such that the X-ray system is a mobile X-ray system. "Carriage" has to be broadly understood, i.e. an element of the X-ray system which carries the X-ray system. In a particular case, the carriage may comprise a stand provided with wheels to be mobile and a more specific carriage of the C-arc mounted (optionally rotatively mounted) onto such a stand. Such a more specific carriage may embed a drive arrangement for translating the C-arc over a horizontal axis.

## BRIEF DESCRIPTION OF THE DRAWINGS

Through the following detailed description of the example embodiments of the present disclosure with reference to the accompanying drawings, the above and other objectives, features, and advantages of the present disclosure will become more apparent. In the drawings, a plurality of embodiments of the present disclosure is explained in a non-restrictive manner by way of examples.

> FIG. 1 illustrates a perspective view of an example motor assembly for C-arm system in accordance with an example embodiment of the present disclosure.
> FIGs. 2A and 2B illustrate a side view and a perspective view of a yoke with a carriage of a C-arm.
> FIG. 3 shows a schematic view of a propelling arrangement according to this disclosure.
> FIGs. 4A-4D respectively illustrate a schematic view, a cross-sectional view, a front view and a perspective view of a harmonic drive.
> FIG. 5 illustrates a power transfer path in a harmonic drive, in an automatic (or motorized) mode.
> FIG. 6 shows a longitudinal cross-sectional detailed view of a yoke assembly mounted to a carriage of a C-arm, comprising a propelling arrangement, a bearing block and a yoke sleeve.
> FIGs. 7A and 7B illustrate a side and cross-sectional longitudinal views of the propelling arrangement of Fig. 6, without the yoke sleeve and without the bearing block.
> FIGs. 8A and 8B illustrate two perspective views of the propelling arrangement of Fig. 7A without the hollow shaft, wherein the yoke-sleeve has been artificially removed (as if it would be invisible) for explanation purpose.
> FIG. 8C illustrates a perspective view of a motor.
> FIG. 9 shows the propelling arrangement of Fig. 6 mounted to an intermediate plate of the carriage.
> FIG. 10 shows a longitudinal cross-sectional view of the shaft assembly of the propelling arrangement of Fig. 7B.
> FIGs. 11A and 11B respectively illustrate a front perspective view and a longitudinal cross-sectional view of the hollow shaft - yoke sleeve interface of the propelling arrangement of Fig. 6.

FIGs. 12A and 12B show an exemplary electromagnetic lock, in respective perspective view and a side view.

FIG. 13 illustrates a power transfer path in a harmonic drive, in a manual mode.

FIG. 14 shows a schematic montage of a potentiometer to a propelling arrangement.

DETAILED DESCRIPTION

The present disclosure will now be described with reference to various example embodiments illustrated in the drawings. It should be appreciated that description of those embodiments is merely to allow those skilled in the art to better understand and further implement example embodiments disclosed herein and is not intended to limit the scope disclosed herein in any manner. It should be noted that similar or same reference signs can be used in the drawings when feasible, and similar or same reference signs can represent the similar or same functions.

As mentioned above, for the C-arm, there is a need to design a compact assembly which might also be easy to assembly and disassembly.

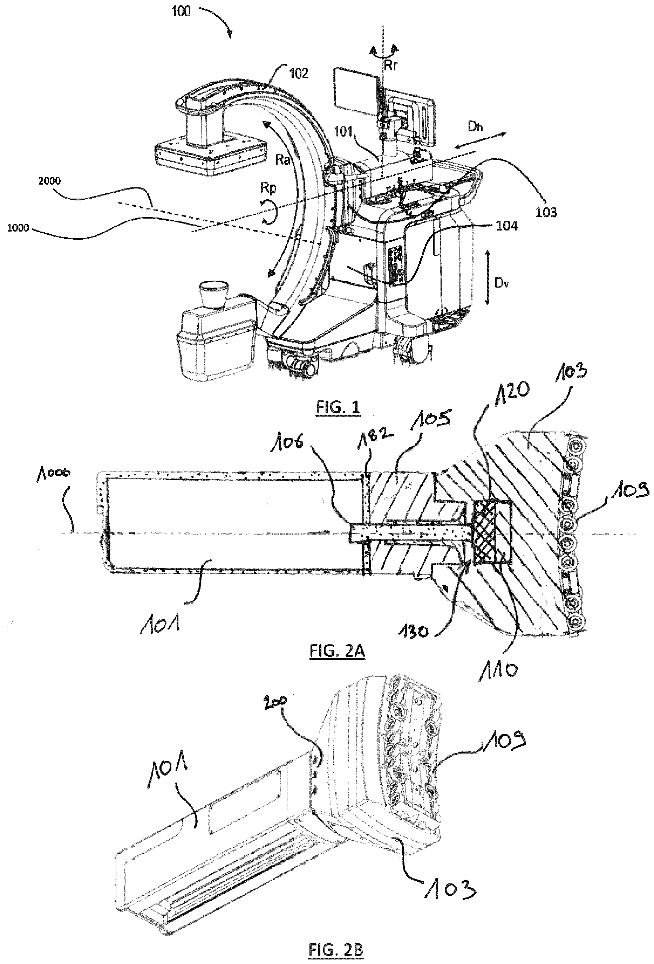

Exemplary embodiments will be described in more detail hereinafter in accordance with Figs. 1-14, wherein Fig. 1 illustrates a perspective view of an exemplary X-ray system, also-called C-arm, 100.

Fig. 1 depicts an exemplary C-arm 100 comprising a stand 104 and a C-arc 102 bearing an X-ray source and X-ray detector on each terminal portion of the C-arc 102. The bottom section of the stand 104 may comprise wheels (or other type of elements of mobility) to provide a mobile X-ray system 100 which can be used in a hospital and medical centers. A carriage 101 is provided between the stand 104 and the C-arc 102. It can be mounted or integral with the stand 104. In a particular case, this carriage 101 is rotationally mounted to the stand 104 about a vertical axis ("Rr"). Additionally or alternatively the stand 104 and/or the carriage 101 may be provided with a vertical drive arrangement so as to vertically elevate the carriage 101 along said vertical axis ("Dh"). The carriage 101 may further embed a drive arrangement for translating in a controlled manner the C-arc 102 along a horizontal axis ("Dh"). As shown in Figs. 1 and 2 the C-arc 102 may be mounted to the carriage 101 via a yoke 103 and a propelling arrangement 130. The yoke 103 is fixedly mounted to the propelling arrangement 130, and the propelling arrangement 130 is rotationally mounted to the carriage 101 so as to drive a propelling movement "Rp" of the C-arc 101 about a horizontal propelling axis 1000. The yoke 103 may further embed (within internal volume 175) an angular arrangement (not referenced) enabling to drive a rotational movement "Ra" about a horizontal angular axis 2000 via wheels or rollers 109, typically orthogonal to the horizontal propelling axis 1000.

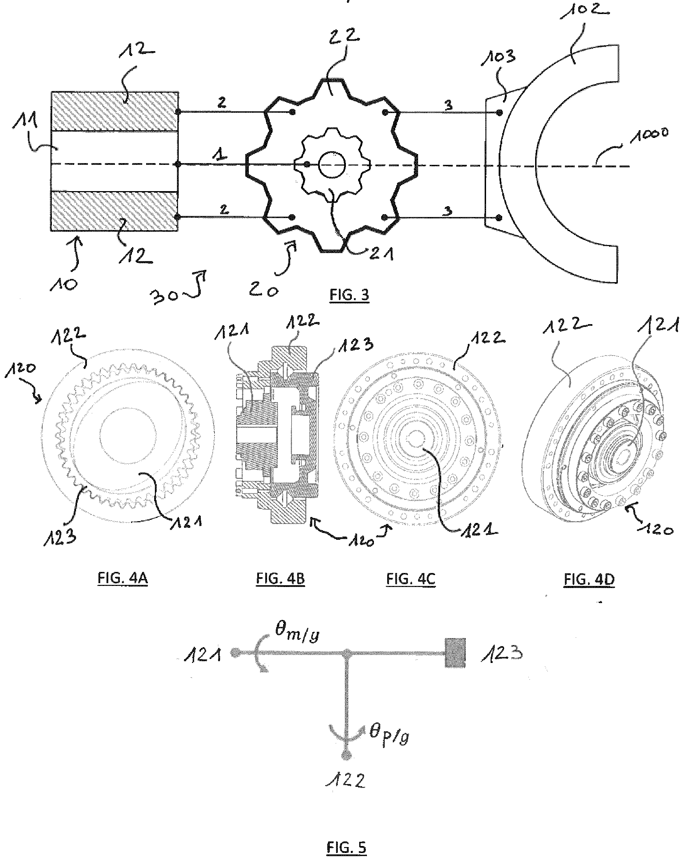

According to this disclosure, and as schematically depicted by Fig. 3, the propelling arrangement 30 to drive the rotation Rp of the C-arc 102 about the propelling axis 1000 (also called "main axis 1000"), comprises:

a motor 10 comprising a rotor 11 and a stator 12,
a transmission mechanism 20 arranged to transmit the motion generated by the motor 10 to the C-arc 102 rotating about the main axis 1000.

The transmission mechanism 20 comprises:

an input 21 to which the rotor 11 is mounted (or fixed) via the mechanical connection 1, the rotor 11 moving according to a rotation Rm in an unlocked configuration,
an output 22 to which the stator 12 and the C-arc 102 are both mounted (or fixed), via the respective mechanical connections 2 and 3, the C-arc 102 and the stator 12 moving according to a rotation Rp.

The transmission mechanism 20, whose input 21 and output 22 are schematically respectively represented by an small gear and a big gear, has a predetermined output/input ratio of transmission, such as for instance equal to or lower than 1/50, or equal to or lower than 1/100, or equal to or lower than 1/150, or equal to or lower than 1/200.

As well-known, any type of mechanical transmission 20, typically using a gearing arrangement, could be used.

As previously mentioned, the C-arc 102 is mounted to the propelling arrangement 30 which is rotationally mounted to the carriage 101. Therefore the carriage 101 can be seen as a stand or a ground reference of the propelling movement driven by the propelling arrangement 30. The montage of the propelling arrangement 30 to the carriage 101 may be implemented via a fixed protrusion (not shown in Fig. 3) extending from the carriage 101 through the propelling arrangement 30. This montage may be made via the transmission mechanism 20 provided with a section or terminal designed for this assembly with the protrusion. In a particular case, said protrusion is a fixed shaft integral with the carriage 101 or fixedly mounted to a fixed plate of the carriage 101 via e.g. a keyless bush as an in-between piece. In also a particular case, a mobile shaft (also not referenced in Fig. 3) may be designed as being said mechanical connection 3 between the transmission mechanism 20 and the C-arc 102. Preferably, the mobile shaft and the fixed shaft are coaxial, one outer shaft being hollow with a lumen for providing the other inner shaft within.

In some exemplary embodiments, the motor 10 may be a brushless or BLDC motor. The rotor 11 can be provided with a motor shaft (not shown) arranged with the input 21 of the transmission mechanism 20 to transmit the rotational movement generated by the motor 10 to the input 21. Preferably a locking mechanism (not shown in Fig. 3) is further provided so as to lock the rotation of the rotor 11, such a locking mechanism being preferably arranged to be remotely triggered such as for instance an electromagnetic lock, such that the propelling movement is in a manual use when the rotor 11 is locked and in an automatic mode (i.e. driven by the motor 10) when the rotor 11 is unlocked. This locking mechanism may be directly or indirectly mounted with the stator 12 (via for instance the yoke 103) such that it rotates together with the stator 12 and the C-arc 102 at the same angular speediness. The C-arc 102 is preferably mounted to the output 22 of the transmission mechanism 20 via the yoke 103 which may further house the motor 10 and the locking mechanism. Such a yoke 103 may be mounted or fixed to an end part of said mobile shaft.

Figs. 4A-D and 5 show a harmonic drive 120 (also called strain wave gear) as an example of a transmission mechanism , such as a harmonic drive 120 may be made by the company Harmonic Drive SE (www.harmonicdrive.de). A harmonic drive comprises a wave generator ("WG") 121 typically having an elliptical cross-section, positioned with an internal flexible spline ("FS") 123 typically provided with external teeth on its outer surface such that FS is deformed or radially moved when WG rotates, to engage with internal teeth provided on internal surface of an outer circular spline ("CS") 122.

The key to the design of such a strain wave gear 120 is that there are fewer teeth (for example two fewer) on the flex spline 123 than there are on the circular spline 122. This means that for every full rotation of the wave generator 121, the flex spline 123 would be required to rotate a slight amount (by two teeth in this example) backward relative to the circular spline 122. Thus the rotation action of the wave generator 121 results in a much slower rotation of the flex spline 123 in the opposite direction. As an alternative embodiment where the flex spline 123 is fixed (mechanically maintained to the ground) and the circular spline 122 is free to rotate, then this is the circular spline 122 to rotate a slight amount (by two teeth in this example) frontward (in this embodiment) relative to the flexible spline 122. Thus the rotation action of the wave generator 121 results in a much slower rotation of the circular spline 122 in the same direction in this alternative embodiment.

The harmonic drive 120 is a two degree of freedom mechanism, similar to planetary gear train. But it has three terminal (WG 121, FS 123 and CS 122) with any two out of the three can be selected as input and output, while the third remains fixed.

In an exemplary embodiment of this disclosure, the C-arm system comprises a harmonic drive having these three terminals which are mounted as follows: WG is the terminal acting as said input 21 and is rigidly connected to the rotor 11, via e.g. a keyless bush.

CS is the terminal acting as said output 22 and is rigidly connected to the C-arc 102 and to the stator 12.

FS is the terminal mechanically rigidly connected to the carriage 101, acting therefore as the fixed ground reference to the propelling arrangement 30.

As a consequence, FS is fixed and the CS rotates in the same rotational direction as the one of the WG (by two teeth for each revolution of the rotor 11, if we consider the above-mentioned example). As indicated by Fig. 5, the wave generator (being said input 21) has a rotation $\theta_{m/g}$ ($\theta$ being the rotation of the motor $m$ with respect to the ground $g$) generated by the rotor 11, the circular spline (being said output 22) has a rotation $\theta_{p/g}$ ($\theta$ being the rotation of the C-arc - propelling $p$ with respect to the ground $g$), the flex spline is fixed and the harmonic drive is defined by its reduction ratio $i$ (e.g. $i$=160). As indicated above, the transmission ratio (or gearing ratio) output/input (or CS/WG) of the harmonic drive depends directly on the difference of number of teeth between the FS and the CS. Note that this is not the gear ratio between the propeller movement and the motor movement, since the stator 12 itself is mounted on the output 22 of the harmonic drive 120, also the output 22 is given to the stator 12 through rotor 11 via harmonic drive 120. Since:

$$\frac{\theta_{p/g}}{\theta_{m/g}} = \frac{1}{i+1} = \frac{1}{161}$$

The effective gear ratio between the rotor 11 and the propelling movement is calculated as follows:

$$\theta_{m/p} = \theta_{m/g} - \theta_{p/g}$$

$$\theta_{m/p} = (i+1) * \theta_{p/g} - \theta_{p/g}$$

$$\theta_{m/p} = i * \theta_{p/g}$$

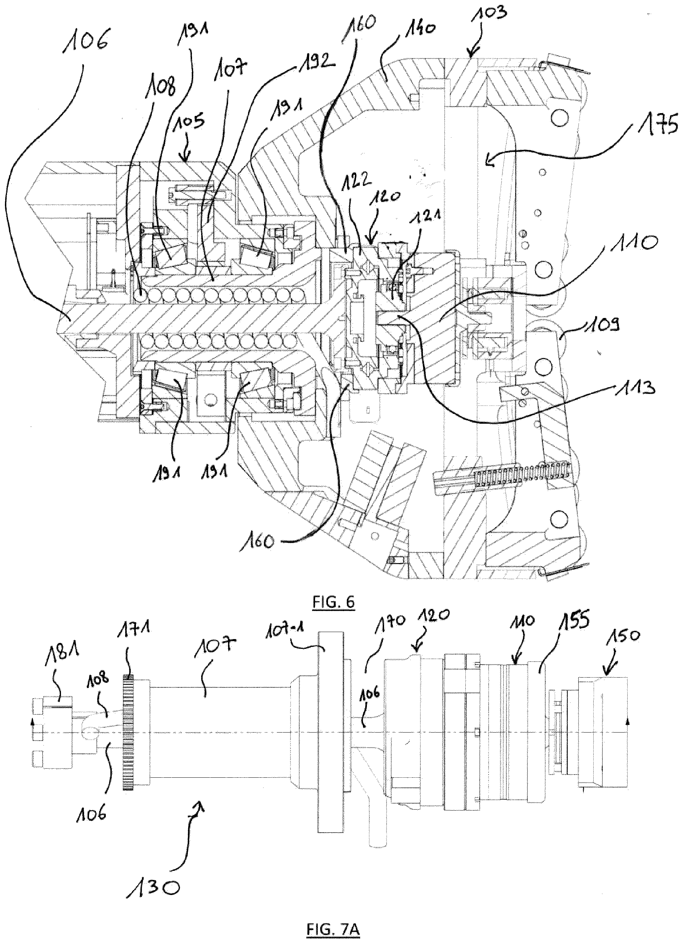

Figs. 6 to 14 show a detailed embodiment of a C-arm according to this disclosure.

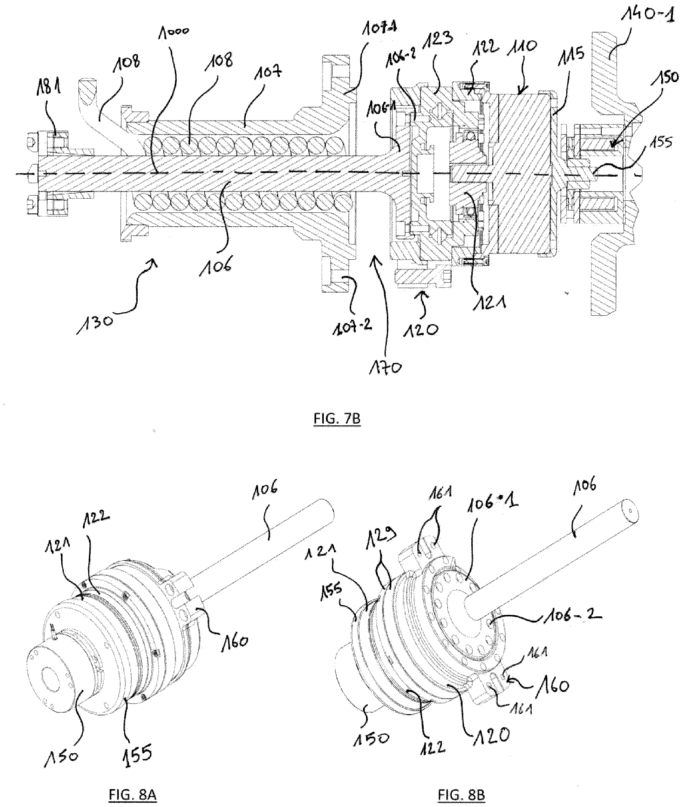

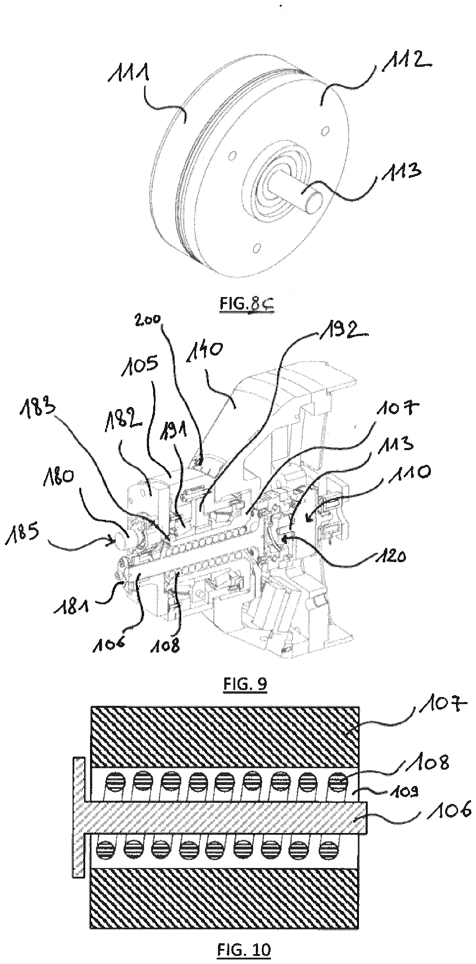

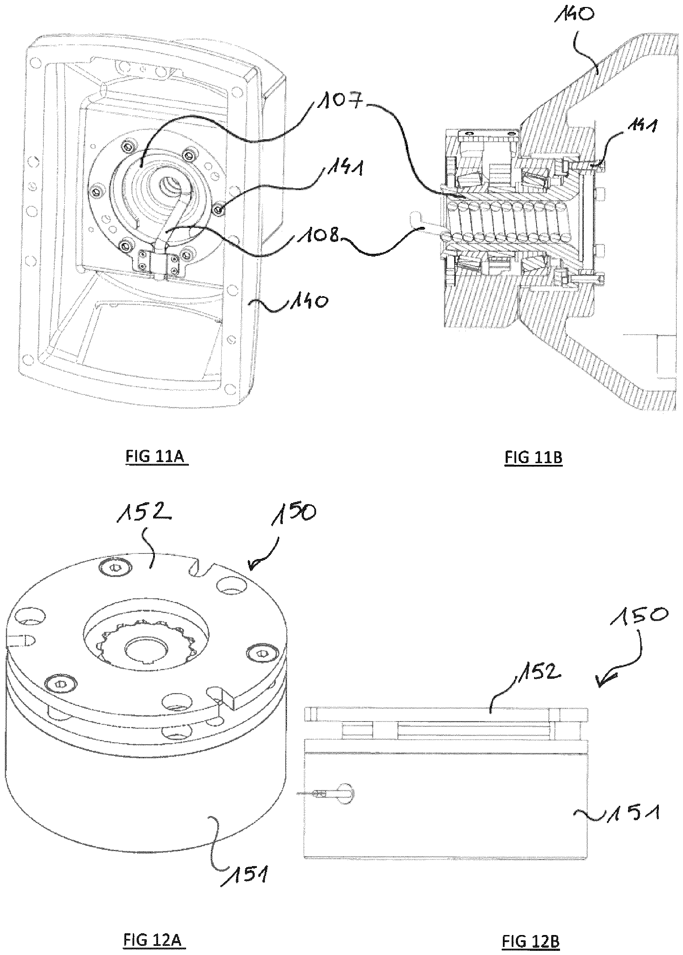

The corresponding propelling arrangement 130 comprises a rotor 111 (optionally comprising a motor shaft 113 extending along the main axis 1000) and a stator 112 of a motor 110, optionally a BDLC motor, respectively mounted (via keyless bush preferably) to the WG (input) 121 and CS (output) 122 of a harmonic drive 120, as above-mentioned. A keyless coupling allows motor 110 to be easily assembled onto or disassembled from the harmonic drive 120. Spacers 129 may be further arranged in-between. The propelling arrangement 130 further comprises a mobile shaft 107 mounted (via a keyless bush preferably) to the CS 120 too at one end, preferably on an opposite side to the side of the CS 120 where the stator 112 is mounted to, and is rotatively mounted to a portion fixed of the carriage 101 at the other end of the mobile shaft 107. This portion fixed to the carriage 101 may be a plate 182 fixed to the bearing block 105 mentioned thereafter provided with a circular slot or a rib in or on which a rib or slot provided at one end of the mobile shaft 107 is rotationally guided (not shown in drawings). Optionally an arcuate shuttle is provided within a slot (not shown in drawings) to provide a rotational stroke higher than 360 degrees (e.g. 400 degrees - i.e. +/- 200 degrees). The sleeve 140 of the yoke 103 of the C-arc 102 is mounted to this mobile shaft 107, via a flange 107-1 extended radially from a segment of the mobile shaft 107 and fixation means 141 (e.g. screws - see Figs. 11A-11B). More particularly a gap 170 (see Fig. 7A) can be provided adjacent to the sleeve 107-1 to arrange in it a complementary ring fixed to or integrally formed in the yoke-sleeve 140 and then fixed to this flange 107-1 (as shown in Fig. 9 or 11B). Then a rotation plate 160 may be mounted on the yoke-sleeve 140 (see Figs. 8A-8B). Preferably this rotation plate 160 is used to mount the hollow shaft 107 - yoke-sleeve 140 assembly to the output 122 of the transmission mechanism 120 (via fixation means - e.g. screws - to fixation holes 161 provided through the rotation plate 160) thus forming the mechanical connection 3 of Fig. 3. Optionally slots or protrusions (not shown) may be provided on the outer surface of the hollow shaft 107 to assist positioning of the yoke-sleeve 140 on it. Furthermore the outer surface of the fixed shaft 106 may be located / aligned with respect to the yoke-sleeve 140 using pins (or slots) on the yoke-sleeve 140 and slots (or pins) on the fixed shaft 106 (not shown). The yoke-sleeve 140 is supported on a bearing block 105 fixed to or integral with the carriage 101 and surrounding a significant portion of the mobile shaft 107. The bearing block 105 has a housing embedding set of (tapered) (roller-)bearing 191 supported on outer surface of the mobile shaft 107. Furthermore a friction pad 192 may be provided within the bearing block 105 in contact with the hollow shaft 107 to better control the movement of the C-arc 102 by restricting movement due to unbalance as further explained below. In addition some spacers may be provided to fill any remaining empty spaces so as to get a final compact and solid assembly and/or to provide motor interfaces (e.g. between the stator 112 and CS). The mobile shaft 107 is hollow and can rotate about a fixed shaft 106, the latter being positioned within the hollow channel of the mobile shaft 107, which is in turn fixed to the carriage 101 via preferably a keyless bush 181 (and an intermediate plate 182 of the carriage 101 as depicted in FIG. 9) on one end of the fixed shaft 106 and to the FS 123 of the harmonic drive 120 on the other end of the fixed shaft 106 (via a potential flange 106-1 extending radially from this end of the fixed shaft 106 - as depicted in Fig. 7B). By using such a keyless-bush 181 the radial play in the mechanism is eliminated. The fixed shaft 106 is therefore a fixed ground reference to the harmonic drive 120. The fixed shaft 106 extends along the propelling axis 1000 (about which the C-arc propels). In that configuration of the C-arc assembly, the yoke 103 is suspended on the (tapered roller) bearings 191. The components in the load path are subjected to significant loads and may be critical from structural safety point of view. The bearings 191 provided between the mobile and the fixed shafts 107, 106 relieve a load about the main axis 1000 and the friction pad 192 compensates a transversal load (transversal to the main axis 1000 - resulting in the abovementioned "unbalance") due to an offset of the center of gravity of the assembly yoke 103-C - arc 102 from the main axis 1000. Electrical cable(s) 108, e.g. spiral cable(s), are optionally arranged in a gap 109 extending between the mobile shaft 107 and the fixed shaft 106, such cable(s) 108 being connected to motor(s) and/or locking mechanism(s) and/or other electrical component(s) of the propelling and/or angular movements, for power supply and/or for data communication and/or feedbacks.

In addition and optionally, this propelling system comprises a locking mechanism comprising an electromagnetic lock ("EM lock") 150 fixed to the assembly stator 111 - yoke 103 - CS 122 - mobile shaft 107, optionally it is fixed to the yoke-sleeve 140 (directly or via an optional attachment element 140-1 fixed to a terminal portion of the yoke sleeve 140). Therefore the EM lock 150 rotates with the yoke 103 about the propelling axis 1000. EM lock 150 comprises a fixed element (e.g. an EM lock housing 151) and a mobile element (a friction disk 152) mobile along the main axis 1000, and is arranged in such a way that the mobile element 152 is in contact with the rotor 111 of the motor 110 when the EM lock 150 is electrically supplied (or not depending on the type of EM lock 150) - locked state - and is not in contact with the rotor 111 when the EM lock 150 is not electrically supplied (or is, depending on the type of EM lock 150) - unlocked state. A lock drum 155 is optionally provided between the EM lock 150 and the motor 110.

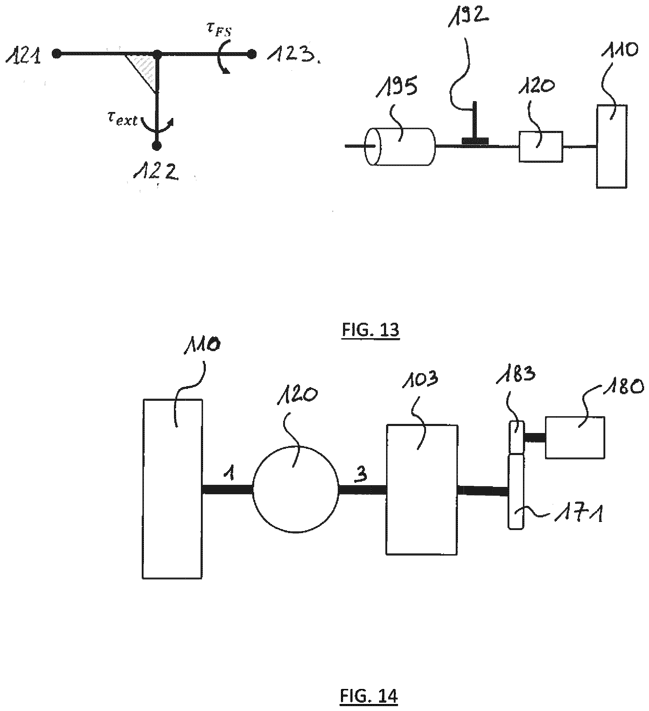

Such EM lock 150 allows the switch between a manual mode in the locked state and an automatic (or motorized) mode in the unlocked mode. It is to be noted that, in the manual mode, wherein the C-arc is moved manually, the output of the transmission mechanism 120 (i.e. the CS 122 of the harmonic drive 120) becomes the input of the transmission mechanism 120 and the input (i.e. the WG 121 of the harmonic drive 120) becomes the output of the transmission mechanism 120 which rotates at the same speediness as the CS 122, the FS 123 being also an output (which ideally should remain fixed). In this manual mode, the fixed shaft 106 (connected to FS) is subjected to maximum torque during the manual mode - which is subject to override condition. Indeed, in the locked state, the (manual) external torque load is mostly transferred to the fixed shaft 106 via harmonic drive 120 (CS 122 to FS 123) and slipped away via the keyless bush 181 of the fixed shaft 106. Fig. 13 illustrates the corresponding power transfer path in the harmonic drive 120, in such a manual mode: there are mostly two loads: the inertial load 195 and the friction load (via mostly the friction pad 192) against which the motor 110 has to do work. In the manual mode, the fixed shaft 106 provides a ground reference to the harmonic drive 120 and connects the flex spline (FS) 123 to the intermediate plate 182 via the keyless bush 181. The fixed shaft 106 is therefore subjected to maximum torque $\tau_{FS}$ during this override condition and the circular spline 122 (attached to the yoke-sleeve 140) acts as an input (manual torque $\tau_{ext}$). Since the EM lock 180 is in locked mode the WG 121 is fixed too. In this locked condition the harmonic drive 120 is therefore completely locked during overriding (or manual use) if lock does not slip. In summary, and as above-mentioned, the external torque $\tau_{ext}$ is directly transferred to the fixed shaft 106 as depicted in Fig. 13.

A potentiometer assembly 185 for the propeller motion may be further fixedly mounted to the carriage 101 (e.g. placed at the back of said intermediate plate 182 fixed to or integral with the carriage 101 as shown in Fig. 9) and arranged with the mobile shaft 107 to measure the angular positions of the C-arc 102. In particular, a gearing 183 of the potentiometer 180 may be meshed with a gearing 171 provided about an end part of the mobile shaft 107, through a hole provided through said intermediate plate. Fig. 14 schematically shows this montage. It should be noted that the potentiometer 180 may be mostly used for homing (or starting the system position). After homing, the position is then preferably tracked by an encoder connected to the motor 110, in case the resolution of the encoder is higher than that of the potentiometer 180.

Propeller position indication markings 200 may be added to the outer surface of the yoke-sleeve 140 and/or to the outer surface of the bearing block 105, to assist the user in angularly positioning the C-arc 102 according to a propelling position. Indication may range from -90° to 0 to 90° as an example.

The following successive steps of a method for dismounting the motor 110 from the C-arm assembly is hereby proposed: Remove:

> C-arc 102,
> yoke-sleeve 140 and holders of the C-arc rollers 109
> EM lock 150
> Keyless bush 181
> Motor 110 connectors
> Rotation plate 160 (from yoke-sleeve 140)
> Motor side spacers 129
> Motor 110 and lock drum 115 from the motor side spacer 129 (motor 110 and lock drum 115 may be an integrated assembly, replaceable together)

With this method, needless to remove the hollow shaft 107 or the bearing block 105 or any other core elements of the propelling arrangement 130, to replace the motor 110. This is clearly an improvement from other types of assemblies. This is mostly due to the fact that most of the core elements of the propelling arrangement 130 are mounted on the transmission mechanism 120.

The following successive steps of a method for dismounting the EM lock 150 from the C-arm assembly is hereby proposed: Remove:

> C-arc 102
> yoke-sleeve 140 and holders of the C-arc rollers 109
> EM lock 150

While the invention has been illustrated and described in detail in the drawings and foregoing description, such illustration and description are to be considered illustrative or exemplary and not restrictive; the invention is not limited to the disclosed embodiments.

For example, it is possible to operate the invention in an embodiment wherein a transmission mechanism is not using a harmonic drive 120, but any other kind of transmission mechanism enabling to apply a propelling speediness to the C-arc 102 modified (preferably reduced) from the rotational speediness at the motor output such as transmission mechanism with only two terminals (one input and one output having connections with rotor, stator and mobile shaft for implementing a system according to this disclosure) such as for instance planetary gears. Furthermore this disclosure is not limited to the above-mentioned arrangement of coaxial shafts, it can use for instance a mobile inner shaft and an outer fixed shaft, and more broadly any other arrangements could be designed as long as the propelling arrangement is mostly hold via the transmission mechanism on one hand and the C-arc 102 is propelled on the other hand with a modified speediness from the motor speediness. Furthermore any type of locking mechanism could be used, alternative to the drum lock above-described, as long as this locking mechanism can lock and unlock the motor or an element moved by the motor. Also the control of the C-arc 102 movement does not necessarily comprise a potentiometer and/or encoder provided with the motor, but any kind of measurements tools that can assist a better control.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. X-ray system (100) comprising:

   a C-arc (102) provided or to be provided with an X-ray source and an X-ray detector;
   a carriage (101) of the C-arc (102) adapted to hold the C-arc (102);
   a drive arrangement (30, 130) to drive the rotation of the C-arc (102) about a main axis (1000) comprising:

   a motor (10, 110) comprising a rotor (11, 111) and a stator (12, 112),
   a transmission mechanism (20, 120) arranged to transmit the motion generated by the motor (10, 110) to the C-arc (102) rotating about the main axis (1000), with a predetermined ratio of transmission, the transmission comprising:

   an input (21) to which the rotor (11) is mounted,
   an output (22) to which the stator (12) and the C-arc (102) are mounted.

2. The X-ray system (100) of claim 1, wherein the transmission mechanism (20, 120) comprises:

   a rotatable shaft (107) mounted on the C-arc (102) and on said output (22),
   a fixed shaft (106) mounted on the carriage (101) so as to fixedly hold the transmission mechanism (20, 120).

3. The X-ray system (100) of claim 2, wherein the rotatable and fixed shafts (107, 106) are coaxial about the main axis (1000).

4. The X-ray system (100) of claim 2 or 3, wherein the rotatable shaft (107) has a lumen extending along the main axis (1000), at least a part of the fixed shaft (106) being arranged within the lumen.

5. The X-ray system (100) of claim 4, wherein the fixed shaft (106) is fixed to the carriage (101) via a keyless bush (181), optionally via an intermediate carrier (182) on which a potentiometer (180) is provided, the potentiometer (180) being engaged with the hollow shaft (107).

6. The X-ray system (100) of any of claims 3 to 5, wherein an electrical cable (108) is provided between the rotatable shaft (107) and the fixed shaft (106), optionally this is a spiral cable (108), optionally the spiral cable (108) is arranged to supply motor (110).

7. The X-ray system (100) of claim 1 wherein the transmission mechanism (20, 120) has a ratio of transmission equal to or lower than 1/100.

8. The X-ray system (100) of claim 1, wherein the transmission mechanism (20, 120) comprises a harmonic drive (120) comprising:

   a wave generator (121) being the input,
   a circular-spline (122) being the output,
   a flex-spline (123) being mounted to the carriage (101).

9. The X-ray system (100) of claims 2 and 7, wherein the flex-spline is mounted to the carriage (101) via the fixed shaft (106).

10. The X-ray system (100) of any of the previous claims, further comprising an electromagnetic lock (180) arranged to lock the motor (10, 110), optionally the electromagnetic lock (180) is a drum brake.

11. The X-ray system (100) of the previous claim, wherein the electromagnetic lock (180) is mounted on the side of the motor (10, 110) opposite to said output of the transmission mechanism (20, 120).

12. The X-ray system (100) of any of the previous claims, further comprising a yoke (103) directly mounted to or integral with the C-arc (102), wherein the drive arrangement (30, 130) is mounted to the yoke (103), optionally together with at least another drive arrangement (30, 130) to drive other movement(s) of the C-arc (102).

13. The X-ray system (100) of any of the previous claims, comprising spacer(s) (129) and/or keyless bush (181) to maintain the drive arrangement (30, 130) in a fixed and stable position within a housing even when the X-ray system (100) is moved.

14. The X-ray system (100) of the previous claim, wherein the carriage (101) is provided with wheels, via optionally a stand (104) holding the carriage (101), to be mobile with respect to the floor.

FIG. 1

FIG. 2A

FIG. 2B

FIG. 3

FIG. 4A   FIG. 4B   FIG. 4C   FIG. 4D

$\theta_{m/y}$

121   123

$\theta_{p/g}$

122

FIG. 5

FIG. 6

FIG. 7A

FIG. 7B

FIG. 8A

FIG. 8B

FIG. 8c

FIG. 9

FIG. 10

FIG 11A

FIG 11B

FIG 12A

FIG 12B

121 •————————————•  123.

$\tau_{FS}$

$\tau_{ext}$

122

192

195   120   110

FIG. 13

110   120   103   183   180

1   3

171

FIG. 14

Europäisches
Patentamt

European
Patent Office

Office européen
des brevets

# EUROPEAN SEARCH REPORT

Application Number

EP 24 15 8826

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/254628 A1 (SALADIN JEAN-PIERRE [FR] ET AL) 17 November 2005 (2005-11-17) * paragraph [0018] - paragraph [0024] * * paragraph [0026] * * paragraph [0029] - paragraph [0035] * * figures 1a-1c * | 1-14 | INV. A61B6/03 A61B6/00 |
| A | US 2017/184190 A1 (KLASSEN JAMES BRENT [CA]) 29 June 2017 (2017-06-29) * paragraph [0005] - paragraph [0016] * * paragraph [0171] * * paragraph [0251] * | 7-9 | |
| A | US 2004/052335 A1 (PILLAI VIPIN J [IN] ET AL) 18 March 2004 (2004-03-18) * paragraph [0002] - paragraph [0009] * | 10-12 | |
| A | US 2023/218251 A1 (HANSROUL MARC [US] ET AL) 13 July 2023 (2023-07-13) * paragraph [0104] - paragraph [0107] * | 5 | |
| A | US 2010/322377 A1 (NIIZEKI RYUICHIRO [JP]) 23 December 2010 (2010-12-23) * paragraph [0089] - paragraph [0101] * * paragraph [0175] - paragraph [0210] * | 1,13 | TECHNICAL FIELDS SEARCHED (IPC) A61B |

The present search report has been drawn up for all claims

| Place of search | Date of completion of the search | Examiner |
|---|---|---|
| Munich | 15 July 2024 | De la Hera, Germán |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 15 8826

This annex lists the patent family members relating to the patent documents cited in the above-mentioned European search report.
The members are as contained in the European Patent Office EDP file on
The European Patent Office is in no way liable for these particulars which are merely given for the purpose of information.

15-07-2024

| Patent document cited in search report | | Publication date | Patent family member(s) | | Publication date |
|---|---|---|---|---|---|
| US 2005254628 | A1 | 17-11-2005 | DE | 102005020024 A1 | 01-12-2005 |
| | | | FR | 2870428 A1 | 18-11-2005 |
| | | | US | 2005254628 A1 | 17-11-2005 |
| US 2017184190 | A1 | 29-06-2017 | US | 2017184190 A1 | 29-06-2017 |
| | | | WO | 2015168793 A2 | 12-11-2015 |
| US 2004052335 | A1 | 18-03-2004 | CN | 1500444 A | 02-06-2004 |
| | | | DE | 60314573 T2 | 06-03-2008 |
| | | | EP | 1397995 A1 | 17-03-2004 |
| | | | JP | 4325792 B2 | 02-09-2009 |
| | | | JP | 2004097828 A | 02-04-2004 |
| | | | US | 2004052335 A1 | 18-03-2004 |
| US 2023218251 | A1 | 13-07-2023 | AU | 2021288678 A1 | 15-12-2022 |
| | | | CA | 3183719 A1 | 16-12-2021 |
| | | | CN | 115768355 A | 07-03-2023 |
| | | | EP | 4164499 A1 | 19-04-2023 |
| | | | JP | 2023529891 A | 12-07-2023 |
| | | | KR | 20230022432 A | 15-02-2023 |
| | | | US | 2023218251 A1 | 13-07-2023 |
| | | | WO | 2021252625 A1 | 16-12-2021 |
| US 2010322377 | A1 | 23-12-2010 | DE | 102010024430 A1 | 17-02-2011 |
| | | | FI | 20105718 A | 23-12-2010 |
| | | | JP | 5567399 B2 | 06-08-2014 |
| | | | JP | 2011025012 A | 10-02-2011 |
| | | | US | 2010322377 A1 | 23-12-2010 |

EPO FORM P0459

For more details about this annex : see Official Journal of the European Patent Office, No. 12/82